(19) **Europäisches Patentamt**

**European Patent Office**

**Office européen des brevets**

(11) **EP 1 307 247 B1**

(12) **EUROPEAN PATENT SPECIFICATION**

(45) Date of publication and mention
of the grant of the patent:
**29.09.2004   Bulletin 2004/40**

(51) Int Cl.⁷: **A61L 31/04**, C08L 89/06

(86) International application number:
**PCT/EP2001/008872**

(21) Application number: **01965155.3**

(22) Date of filing: **01.08.2001**

(87) International publication number:
**WO 2002/009790 (07.02.2002 Gazette 2002/06)**

(54) **COLLAGEN MEMBRANE ARRANGED AT MACROMOLECULAR LEVEL**

KOLLAGENMEMBRAN MIT MAKROMOLEKULARER ANORDNUNG

MEMBRANE DE COLLAGENE DISPOSEE AU NIVEAU MACROMOLECULAIRE

(84) Designated Contracting States:
**AT BE CH CY DE DK ES FI FR GB GR IE IT LI LU
MC NL PT SE TR**

(30) Priority: **02.08.2000  IT   MI001794**

(43) Date of publication of application:
**07.05.2003   Bulletin 2003/19**

(73) Proprietors:
• **Mediolanum Farmaceutici S.p.A.
20143 Milano (IT)**
• **OPOCRIN S.p.A.
41040 Corlo (MO) (IT)**

(72) Inventor: **PARMA, Bruna, c/o Opocrin S.p.A.
I-41040 Corlo (IT)**

(74) Representative: **Gervasi, Gemma, Dr. et al
NOTARBARTOLO & GERVASI
Corso di Porta Vittoria, 9
20122 Milano (IT)**

(56) References cited:
**WO-A-00/29484          WO-A-95/18638
WO-A-98/44809          US-A- 5 785 983**

**Description**

FIELD OF THE INVENTION

The technical field of the present invention concerns the preparation of biocompatible supports particularly consisting of collagen membranes for tissue reconstruction.

PREVIOUS ART

Collagen is a scleroprotein present in different ways in animal tissues and organs.

In humans it is one of the most represented structural proteins and constitutes approx. 30% of the total proteins.

Various collagen types exist which differ mainly with respect to the function of the tissue or organ of origin. Ample literature is available for all collagen types relating to primary, secondary structure, composition, purity, uses (Marcel E. Nimni *Collagen Biochemistry, Biochemistry and Biomechanics, Biotechnology,* CRC Press Inc. Boca Raton Florida 1988; Cunningham and Frederiksen *Methods in Enzymology,* Academic Press, New York, 1-554,1982).

It is known that the properties of collagen do not depend solely on the structure of proteic monomers making up the triple helix of the fibre, but, above all, on the complex macromolecular organization that distinguishes this and other structural proteins with mechanical functions (e.g. myosin and tropomyosin in muscular fibres, etc.) and that envisages organization in fibrillae, their supercoiling, their organization in head-tail monomers and so on.

The macromolecular organization of the native collagen structures, is partly lost with the collagen extraction processes. It can be partly restored artificially, for instance by chemical cross-linking. This treatment restores, even if only partially, the mechanical resistance of the collagen fibrillae and makes the end product also more resistant to proteolytic digestion.

So far many collagen based preparations have been introduced into medical practice alone or with other natural components, both in gel form and feltwork, membranes or plasters, as stimulating agents for the cicatrization of wounds, or as vehicles or devices for the slow release of other drugs or as matrices suitable for interaction with cells.

The importance of collagen as a substrate suited to favouring interaction and cellular growth is in fact known. Also known is the preference of cellular lines of different origin for the various collagen types: for instance skin fibroblasts and endothelial epithelial cells (Murray J.C. et al. Cancer Res. 40,347, 1980; Wicha M.S. et al. Exp. Cell Res. 124,81, 1979) grow better on type IV collagen (a collagen type present in the basal membrane) while chondrocytes prefer the type II collagen (present in the hyaline cartilage) (Hewit A.T. et al. Proc. Natl. Acad. Sci. USA 77,385, 1980) but they also adapt to grow on collagen I and III.

In this type of application the presence of fibronectin, endogenously produced by the cells (Grinnel F. et al. Proc. Natl. Acad. Sci. USA 75,4408 (1978), also proved important for the adhesion of fibroblasts on the collagen substrate (Pearlstein E., Int. J. Cancer 22,32, 1978) and that of chondronectin for the interaction of chondrocytes with the collagen (Hewit et al. Proc. Natl. Acad. Sci. USA 77,385, 1980).

In the literature a number of collagen applications and extraction and preparation methods are available.

The US patent 5,785,983 claims a preparation process of type I collagen membranes, chemically unmodified, obtained from collagen gel distributed in non-stick Teflon trays, by slow exsiccation under suitable conditions of temperature, vacuum and nitrogen flows. The membranes thus obtained are proposed in the cicatrization of wounds and burns and as interposition barriers to prevent adherences after internal surgery operations. The preparation of collagen gel from bovine tendon is described in WO98/44809.

Many patents, such as EP 284789, exist on feltwork or collagen membranes obtained by freezing and rapid lyophilization of collagen solutions in gel form. The structure of such feltwork is not organized since such processes fix, as in an instant photograph, the disorder of the collagen fibres in solution. As said in EP 284789, the structure obtained is not suitable to allow cell growth. In the literature is described how the pore size of such feltwork is regulated by controlling the temperature of freezing, However, the resulting pores have a diameter exceeding that of cultured cell, which are allowed to pass through it (Boyce et al. J. Biochem. Mat. Research, 1988 22:939-957). Moreover, these membranes are of limited resistance since the collagen, denatured in the extraction process, is not sufficiently reorganized.

The patent WO95/18638 claims reabsorbable collagen membranes, as a guide in the repair of tissues and characterized by two opposite sides, a fibrous one suitable for the growth of cells and a smooth one to inhibit adherences. Such membranes are obtained from peritoneal or placental membranes from various mammals, among which calves, and are prepared by a rough process of cleaning, dehydration and degreasing with acetone and N-hexane. Due to the preparation method used, the membranes have the shape and the dimensions of the organ from which they originate and also the distinctive characteristics, namely porosity and the collagen type of which they are made up, closely reflect those of the starting tissue.

The patent WO 96/25961 claims reabsorbable matrices of type II collagen (from cartilage) suitable for natural cartilaginous tissue reconstructions. Such matrices are prepared by freezing and/or lyophilization and can be combined with glycosaminoglycans

The patent WO99/19005 claims a multilayer membrane including a matrix mainly of collagen II consisting of a spongy side and a relatively impermeable side. This membrane is prepared by successive stratifications of collagen matrices (previously degreased with acetone), and portions of collagen gel are then lyophilized on such membranes in successive and alternate cycles.

So far, the preparation processes of the collagen membranes envisage the extraction of collagen in semi-purified solid or gel form that is quickly frozen and dried. Generally, the collagen is subsequently chemically modified by cross-linking, occurring in a random way without macromolecular reorganization typical of the native fibrilla, to increase the mechanical resistance of the membrane.

Alternatively, the membranes can be prepared by slow evaporation of a collagen gel, or directly prepared from collagen membranes pre-existing in nature and only partially purified.

One unsolved technical problem which still remains is to produce collagen membranes in which the collagen is arranged at macromolecular level in a more similar way to its natural form, which are endowed with mechanical resistance and elasticity, though being mouldable, according to the requirements of the reconstruction site.

SUMMARY

The present invention concerns double sided collagen membranes with a porous side suitable for the adhesion and/or growth of cells and a smooth side, characterized in that the collagen on the smooth side is arranged at a macromolecular level in a similar way to the native one. The collagen comprising such membranes is of type I, II, III or IV. An additional subject of the present invention is a process for the preparation of collagen membranes characterized in that a collagen gel is strained and desiccated very slowly on plates or trays on which an electrostatic charge has been induced, and the use of the collagen membranes as supports for the adhesion and/or growth in vitro and in vivo of mammalian cells, particularly in the applications of tissue reconstruction (for instance of epithelial, bone, cartilaginous or connective tissue) matrix-guided or induced.

DESCRIPTION OF THE FIGURES

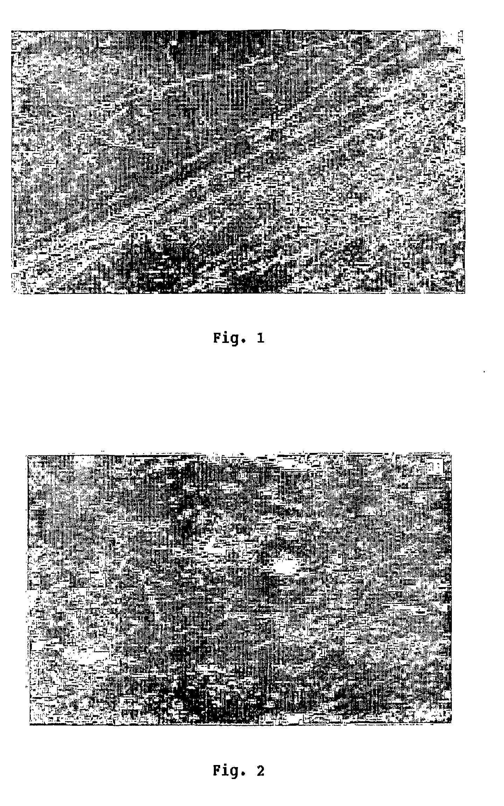

**Figure 1. Smooth side of the membranes as seen under the Scanning Electron Microscope (SEM).**
The sample was treated as follows: 2 small portions of the membrane produced according to the invention (approx. 5 x 3 mm) were cut out from each sample. These were placed on a special metallic support (stub) exposing the smooth side for each sample (A, B and E). The samples were covered with a palladium gold film to render its surface conductive and allows its observation under the SEM. SEM analysis of the shiny surface of the samples highlighted the presence of fibres arranged at macromolecular level in the smooth part.

**Figure 2. Porous side of the membranes as seen under the Scanning Electron Microscope (SEM).**
The sample was treated as follows: 2 small portions of the membrane produced according to the invention (approx. 5 x 3 mm) were cut out from each sample. These were put on a special metallic support (stub) exposing the opaque part (marked with a light incision on the support) for each sample (A, B and E). The samples were covered with a palladium gold film to render its surface conductive and allow its observation under the SEM.
SEM analysis of the opaque surface of the samples highlighted the presence of superficial irregularities, protuberances and "bubbles" which confer a certain porosity to the membrane.

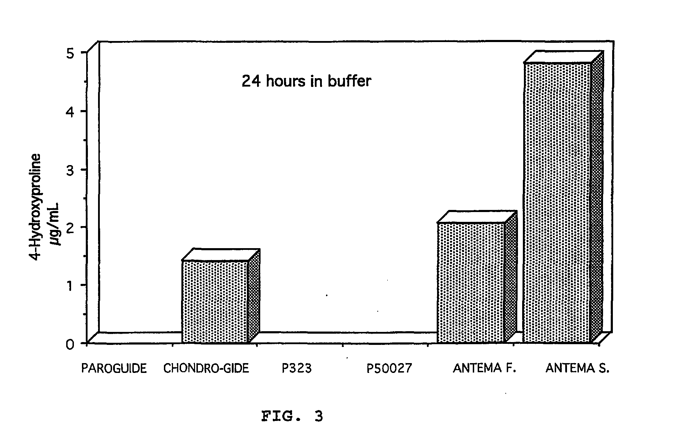

**Figure 3. Resistance of the membranes to digestion with collagenase.**
Membranes produced according to the present invention (batch P323 and P50027) and commercially available membranes (ChondroGide, Antema F and Antema S) were subjected to enzymatic digestion with collagenase. The content of free. hydroxyproline, an indication of enzymatic digestion, was measured in the samples after 24 hours: the membranes according to the present invention proved more resistant after such period.

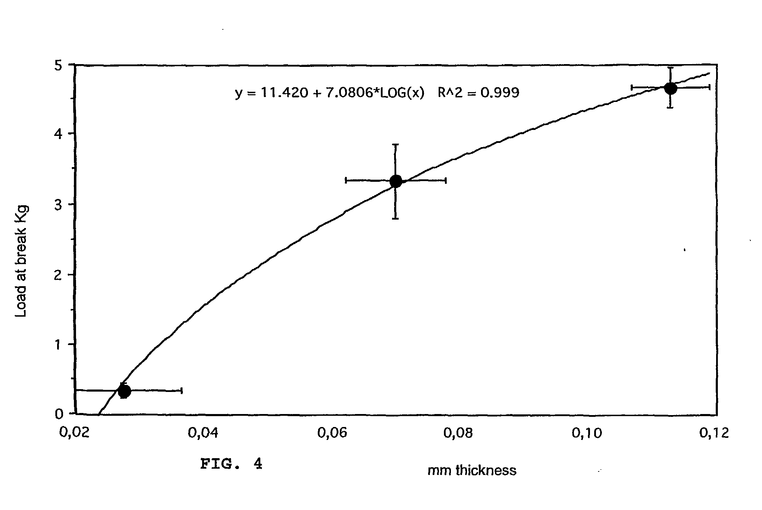

**Figure 4. Mechanical resistance curve of the membranes according to the present invention.**
Small dog bone shaped samples (maximum length 76.7 mm and the thinnest part 4 mm wide and 22 mm long) were cut from the membranes produced and subjected to tensile tests. according to conventional processes, with Chantillon apparatus HTC mod. (USA). The mean resistance values measured in Kg of ultimate tensile strength for 3 families of samples (3-6 samples for each family) of different thickness, were displayed in graph form. The relationship between mean thickness (x), in mm and mean load at break ultimate tensile strength (y) of the membranes is described by the logarithmic equation: $y = 11.42 + 7.08 \log x$, with a correlation index $R^2 = 0.999$.

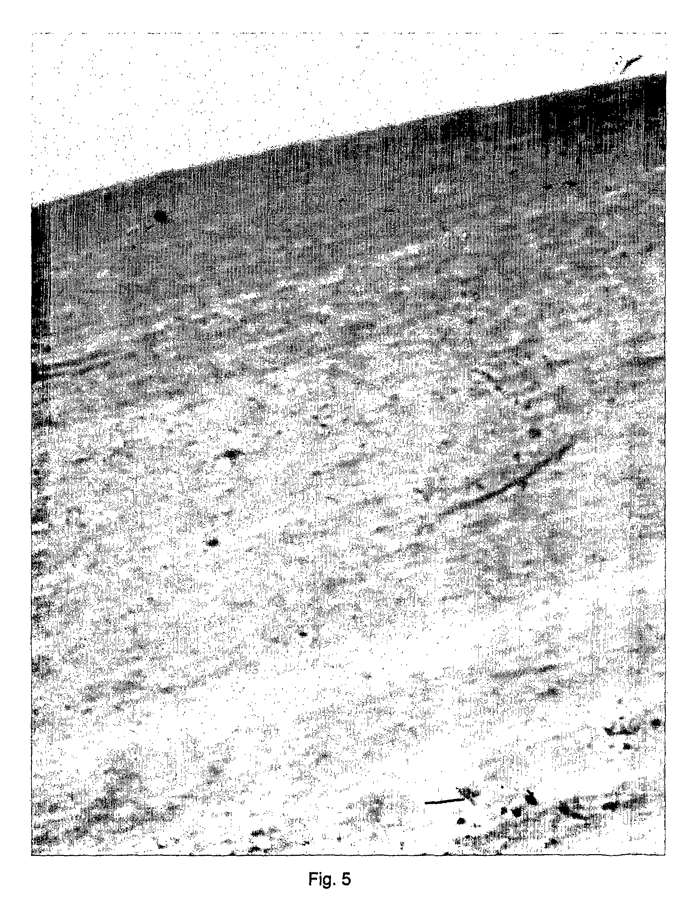

**Figure 5. Optic microscopy of the collagen membranes prepared according to Example 7.**

Semi-fine sections, perpendicular to the membrane surface, of the 40046 membrane were stained with basic dyes of the thiazine group and observed under the optic microscope. One surface (the upper one in the figure, corresponding to the side in contact with the electrostatically charged side of the tray during the dry phase) is smooth, compact and intensely coloured. Approaching toward the opposite surface, which corresponds to the lower surface in the picture (which is the one exposed to the air during the drying phase) the structure becomes is less homogeneous, more loose and uneven. Enlargment 50X.

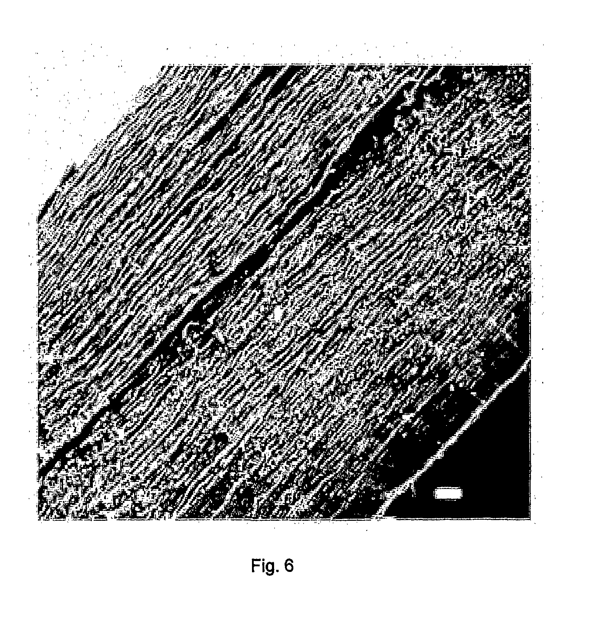

**Figure 6. Collagen membranes batch 40046, seen at the electron microscope.** Semi-fine sections of the collagen membranes prepared according to example 7, perpendicular to the membrane surface, were prepared according to Maunsbach AB and Afzelius B. 1999, Academic Press. It can be observed that the lower part of the picture, corresponding to the side in contact with the air during the dry phase (opaque side) is irregular, while the opposite side in contact with the electrostatically "charged tray" (corresponding to the upper part of the figure, is more regular and the collagen fibres are more compact and arranged in the longitudinal direction

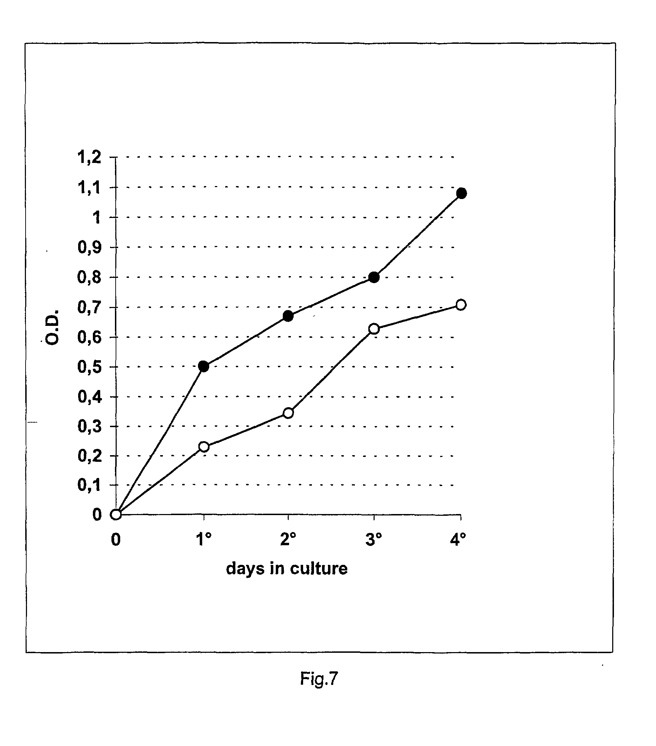

**Figure 7. Growth of dermal fibroblasts on collagen membranes 40046 compared growth in cell-culture treated plastic wells.** The growth of dermal fibroblast on collagen membrane prepared according to example 7 (40046) was compared to the growth on cell-culture treated plastic wells, as measured by the MTT test, as described in example 13. Fibroblast on collagen membrane (-○-) and fibroblast on plastic wells (-●-). O.D. optical density.

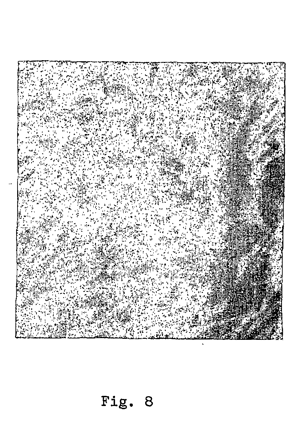

**Figure 8. Dermal fibroblasts after 6 days of growth on collagen membranes observed by confocal microscope.** Dermal fibroblasts were plated on collagen membranes prepared according to the invention and after 6 days were stained as described in example 14. Cells were allowed to reach confluency. Microscopy observation showed that cells grew at different levels in the membrane. At day 6, the fibroblasts, of polygonal shape and elongated, were at confluence as they covered all the membrane space (labeling with Phalloidine) Obj: 10.0 Zoom: 1.430.

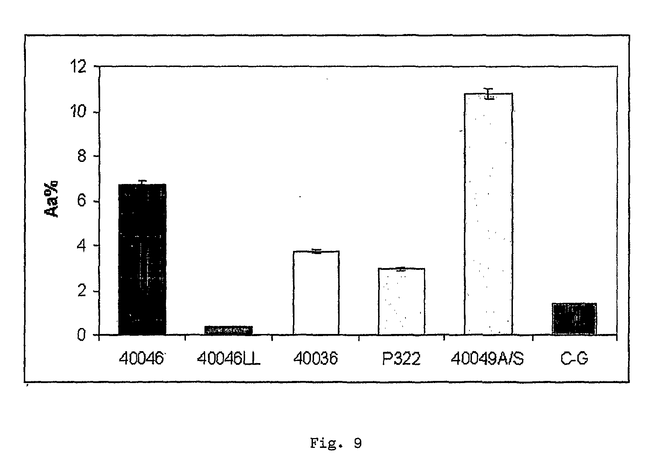

**Figure 9. Histomorphometry of collagen membranes according to the invention and commercial collagen membranes.** Human chondrocytes (1x104) were allowed to grow for 7 days on collagen membranes produced according to the invention (40046, 40036 and 40049A/S) or according to the invention but lyophilized (40046LL), or on commercially available membranes (P322 and C-G). At the end of the 7 days period, the membranes, which were colonized by cells at a different extent were stained with Safranine-O, which is specific for the extracellular matrix sulphate groups, as described in example 16. Since only vital condrocytes synthetize cartilagineous matrix, a positive safranin staining indicates that the cells are alive and didn't loose their ability. The histomorphometry data were obtained by plotting for each membrane the percent of the entire surface which was colonized by chondrocytes after a 7 days period (Aa %).

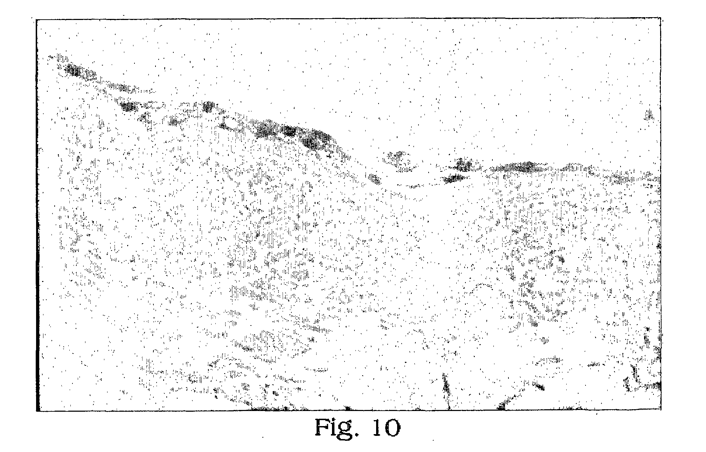

**Figure 10. Microscope analysis of a commercially available collagen membrane (ChondroGide).** Chondrocytes grown on a commercially available membrane were stained with safranin-O. Cells are few, elongated and not intensely coloured with the dye, indicating a low ability to synthetize the extracellular matrix. They are arranged in a single layer on the membrane.

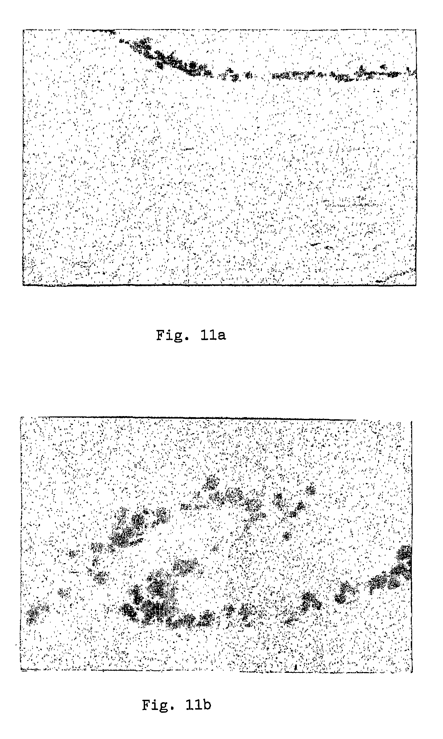

**Figure 11. Microscope analysis of the 40046 membrane produced according to example 7.** a) Chondrocyte grown on membrane 40046 were stained with safranin-O. Chondrocytes grown on 40046 membrane are morphologically very different from the ones showed in figure 10: they are rounded, intensely coloured because of the presence of abundant sulphate groups and they grow in multilayer, showing the ability to penetrate into the membrane. b) Enlargement of a particular.

DETAILED DESCRIPTION OF THE INVENTION

Object of the present invention are double sided collagen membranes, with a smooth side and a porous one, the former organized at the macromolecular level and the latter suitable for the growth of mammalian cells. Such membranes are characterized in that the collagen is arranged at macromolecular level on the smooth side and the fibrillae are organized in the bidimensional space. The membranes of the present invention are endowed with a high mechanical resistance and are also slowly reabsorbable, therefore proving useful in applications of *in vivo* tissue reconstruction. One additional aspect of the invention in fact concerns the use of collagen membranes for tissue reconstruction: these are in fact biocompatible and are colonized *in vitro* by mammalian cells preferably chosen from: staminal cells, fibroblasts, epithelial cells, endothelial cells, osteocytes, chondrocytes.

The membranes of the present invention mainly consist of collagen of type I, II, III, or IV, or mixtures of at least two of the different collagen types. They have a collagen content of between 70% and 95%, measured as dry collagen residue and corresponding to a total hydroxyproline content of between 9% and 13% and a water content not exceeding 15%. They can comprise other constituents, be for instance natural substances and biological components of specific body districts "areas" or characteristics of particular organs or particular functions, such as, for instance, hyaluronic acid or glycosaminoglycans in various percentages.

The membranes of the present invention are not immunogenic, since the telopeptides that constitute the terminal part of the collagen fibrilla are removed by enzymatic digestion with protease: the collagen constituting such membranes is therefore also called as atelocollagen (collagen without the terminal telopeptides).

An additional embodiment of the present invention is the process for the preparation of such collagen membranes, characterized in that a collagen gel is poured into electrostatically charged trays or plates and subsequently slowly dried, for a time exceeding 40 hours, or until obtaining membranes with a water content not exceeding 15%. Exsiccation of the collagen gel preferably takes place at a temperature of between 10 and 40°C, preferably between 20 and 30°C, still more preferably at 26°C, in a turbulence free ventilated incubator, namely an incubator with diaphragmed air flow intake.

After exsiccation, the membranes were sterilized by physical treatment, for instance radiation with γ rays at a dose of between 2.5 and 25KGy.

The trays or plates are electrostatically charged according to method known in the art i.e. by rubbing them with suitable materials for example, or preferably by placing the trays between the plates of a condenser. The induction of an electrostatic charge on the trays can be checked by an Ionizing Fan (RPO Electronic snc, Milan) working at 220 primary volt, secondary volt 6000 VA power 50 Hz 50/60. The trays are of standard shape or of suitable shape and size to give the membrane a shape fit for the subsequent use.

Alternatively, the collagen gel can be poured on trays subjected to an electric field set in a suitable way, maintained if necessary also during the exsiccation step of the gel.

The induction of an electric field or an electrostatic charge allows, surprisingly, and unlike the known art, the macromolecular reorganization and orientation of the collagen into fibrillar structures similar to those of the native tissue, which allow the attainment of membranes endowed with maximum resistance and tenacity.

The gel used in the process, according to the invention, consists essentially of collagen at a concentration of between 0.5 and 6% (weight/volume, where the weight is denoted as dry collagen residue), preferably at a concentration of between 1% and 4%, still more preferably between 2% and 3%. Various natural and non-natural substances can be optionally added to the gel, or biological components of specific district of the organism or of particular tissues. An example of biological components which can be added are animal or vegetable polysaccharides or matrix proteins namely fibronectin, laminin, etc.

In one of its preferred embodiments, the process for the preparation of collagen gel essentially comprises the following steps:

a) mechanical mincing of a connective tissue and homogenization in a collagen suspension,

b) treatment of the collagen suspension with proteolytic enzymes in aqueous solution at acidic pH, optionally followed by filtering,

c) alkaline treatment of the suspension until a pH higher than 8 and its neutralization with acids up to a pH preferably comprised between 5 and 6

d) precipitation of the collagen fibers by addition of an acid solution (salting out)

e) resuspension of the collagen precipitate at a concentration comprised between 0.5 and 6% (weight/volume) of dry collagen residue, in a slightly acidic solution such as an aqueous solution containing a weak acid, and up to obtain a collagen gel in acidic solution. According to a particularly preferred embodiment, two additional steps are added in between step d) and e), which are respectively the steps d') of: resuspension of the precipitate in a strong alkaline solution with mixing for a time of at least 30', and of d") precipitation of the collagen fibers by salting out of the alkaline solution (slowly adding an acidic solution) to pH values comprised between 5 and 6. These two additional steps, which comprises a second precipitation of the collagen fibres, allow for a better purity of the collagen gel and, consequently, of the membranes produced by this gel. The membranes according to these additional steps showed also to be more elastic.

According to the process of the present invention the starting connective tissue is preferably obtained from mammals. A cartilaginous tissue is used, such as that of the trachea, in the case in which one wishes to obtain membranes mainly consisting of type II collagen, or a connective tissue is used that contains mainly type I collagen, namely tendon, preferably derived from equine, bovine, swine animals, or connective tissue derived from the basal lamina, that mainly contains type IV collagen. The final type of use of the membrane determines the most appropriate choice of starting tissue according to what is known in the art and also in consideration of the amounts required. Other sources of collagen can be used as an alternative to mammalian connective tissue, for instance cartilaginous or osteo-cartilaginous tissue of fish.

The mincing of the starting connective tissue (step (a) of the process) is obtained by treatment of the tissue, to which water or an aqueous solution is added, preferably in an amount equal to 6-30 volumes of the initial tissue volume, with a rotating blade mixer until a collagen suspension is obtained. The collagen suspension contains collagen particles preferably less than 25-30 mesh in size.

Other systems of mincing or homogenization are known to the person skilled in the art and can therefore be used as alternatives to present step a). A further reduction of the collagen particles, after homogenization, is preferred and is carried out by treating the collagen suspension with a micronizer, for instance the Waring Blender at 15-20,000 revs./min pulses.

The suspension thus obtained is acidified and treated (step b of the process) with proteolytic enzymes, of non-collagenasic kind, preferably with pepsin, at acidic pH, comprised between 2 and 3, preferably 2.5. Methods for proteolytic digestion and enzyme:collagen ratio are known in the art and may be adjusted accordingly. The proteolytic treatment has the double effect of degrading possible contaminating proteins and of removing the telopeptides of the collagen, which are strongly immunogenic. The collagen obtained is atelocollagen, since it lacks the terminal immunogenic telopeptides. Treatment with pepsin is preferably performed for a period of time comprised between 10 and 20 hours, still more preferably 16 hours and is accompanied with agitation for the first 2-4 hours.

Optionally the collagen suspension can be filtered, through sieves, with a porosity preferably comprised between 20 and 100 mesh, preferably at 25 mesh.

The suspension is subsequently treated with alkali according to step (c) preferably with a concentrated solution of a strong base, preferably NaOH, up to a pH exceeding 8, preferably more than 10. This is achieved by alkaline treatment which has the double effect of removing possible contaminating agents and, partially, the glycosidic residues.

The pH of the collagen suspension is therefore neutralized with a solution of a strong acid, up to a pH of between 5 and 6, preferably a pH of approx. 5.5. The alkalinization and the successive neutralization, (step (c) of the process), are preferably very gradual to avoid a massive precipitation of the collagen fibres.

Alternatively, as in the case in which strong alkalinization of the material is unnecessary, or in other cases known to the person skilled in the art, the pH can be brought to values comprised between 5 and 6, directly after the enzymatic digestion at acid pH with a strong base.

At a pH of comprised 5 and 6 the collagen precipitates by salting out as a whitish mass that can be collected and separated. According to a preferred embodiment, this whitish mass is resuspended in a strong alkaline solution, such as NaOH 1N and left under mixing for 1 hour. After that, collagen is precipitated by addition of an acid solution (salting out) at pH values comprised between 5-6. A collagen gel is therefore obtained by resuspending the collagen precipitate at a concentration (denoted as a percentage in weight of the dry collagen residue) comprised between 0.3 and 6%, preferably between 0.5 and 4%, still more preferably 2%, preferably after removal of the precipitation salts by washing with sterile distilled water, in a solution diluted with a weak acid, preferably acetic acid at a concentration comprised between 0.1 and 1% (w/v), preferably 0.3%. As an alternative to acetic acid, other weak acids, for instance citric acid, ascorbic acid or tartaric acid, at a concentration comprised between 0.5 and 5%, can be used. This solution can be optionally further homogenized in order to help the resuspension of the precipitate. After resuspension, the collagen solution is preferably degassed.

It is then poured very slowly into electrostatically charged trays or plates, as described above, of suitable shape and dimensions, in amounts corresponding to the desired membrane thickness, considering the loss in volume linked to exsiccation. The membranes thus obtained are then sterilized by physical methods for example, namely irradiation with γ rays at a dose of between 2.5 and 25 KGy. In one of its further embodiments of the invention concerns collagen membranes obtainable according to the processes described: namely the one which comprises the pouring of a collagen gel into electrostatically charged trays and the one which comprise the preparation of such gel according to steps a)-e) and a)-e") of the process, as previously described. The collagen gel can be also mixed with natural substances and/or biological components of specific tissues or organic areas (e.g. glycosaminoglycans, hyaluronic acid etc.), before pouring into the electrostatically charged plates to obtain mixed composition membranes as close as possible to the specific natural composition of the tissue to be repaired.

In a further embodiment of the invention concerns the use of the collagen membranes according to the invention, as supports for the adhesion, colonization and/or growth of mammalian cells of various origin, e.g. staminal cells, fibroblasts, epithelial or endothelial cells, chondrocytes, osteocytes, both for *in vitro* systems, and *ex-vivo* systems, namely those where the cells of the patient are removed and put in culture *in vitro*, where they are amplified or simply made to adsorb or to adhere to the membrane. Therefore, in a further embodiment these membranes are for use in therapy. In particular they are used either colonized or not by autologous or heterologous cells, for the matrix guided tissue reconstruction, of tissues namely cartilaginous or osteo-cartilaginous tissue, epithelial or endothelial tissue or connective tissue. For the purposes of the present invention the terms matrix or support are used indifferently, simply defining both a physical framework that the cell uses to both adhere and/or grow also tridimensionally, as well as a substrate capable of interacting with the cellular mechanisms of adhesion, colonization or proliferation. In the case in which the collagen membranes, according to the present invention, are used as matrices or supports for the adhesion, colonization or growth of cells, the membrane becomes a cellular matrix, useful for the matrix guided tissue reconstruction purposes.

EXPERIMENTAL PART

**Example 1. Preparation of collagen gel from cartilage**

Cartilage containing mainly type II collagen, derived from bovine trachea was previously degreased mechanically, frozen at -20°C and broken up into fragments of approx. 2- 3 cm of diameter. 56 g of such fragments was placed in 900 ml of distilled water and around in portions in blender model PBI 16 at 7400 rpm

The white fibres obtained were put in 15 litres of demineralized water, brought to pH 2.51 with 30% HCl and added with 0.588 g of pepsin (700 FIP-U/g- 19917 Merck). The mass was kept under agitation for 2 hours and the pH was kept at 2.5 with HCl.

After a one night rest the fibres, separated by decantation, were homogenized in the blender at 6800 rpm, filtered through a sieve with dimensions ≤ 25 mesh and added to the mother-waters. A solution of 15% NaOH was added up to pH 5.5.

The collagen precipitate was separated and washed with 2 volumes of water at pH 5.5. 107.1g of damp fibres were obtained to which 311.6 ml of distilled water and 0.93 g of glacial acetic acid were added.

In these conditions the collagen absorbed water and assumed a jelly form. The gel was homogenized in blender model PBI 16 at 8400 rpm and was observed to have a dry residue of 4.51%. 310 ml of water were then added and 621 ml of gel were obtained by homogenization having a dry residue (DR) of 2.03 % and corresponding to a 2% collagen gel.

**Example 2. Preparation of collagen gel from horse tendon.**

50 g of horse tendon, corresponding to a dry residue of 35.5% were put in 900 ml of $H_2O$ for 4 h and manipulated to break the granules. They were then transferred into 15 L of $H_2O$ brought to pH 2.5 with HCl in which 1.5g of pepsin were dissolved (Merck 700 U. FIP/g) and enzymatic digestion was allowed to proceed for approx. 20 h at approx. 15°C.

The larger tendon fragments were eliminated by filtering on sieves of 25 mesh. The suspension was then brought to pH 5.4 with commercial NaOHand the collagen precipitate collected in approx. 2 h. The precipitate was collected by decantation and 7.5 L of water at pH 6 were added.

The fibres were washed by agitation, left to rest for 1h and again removed by decantation. Washings were repeated all 3 times. 43 g of damp fibres were obtained from which, by addition of 0.376g glacial acetic acid, 82 ml of water and homogenization, were obtained 125 g of collagen gel having 2.45% dry residue.

**Example 3. Large-scale preparation of collagen gel.**

500 g of horse tendon and 10 L of distilled water, were subjected to mashing by Rayneri turbotest SB endowed with sharp, curved blades rotating at 1500 revs./minute then with Rayneri turbotest H 002 then with INOX Waring blender micronizer at a speed of 15-20.000 rpm/min with pulses.

The suspension was left to rest until swelling (approx. 2- 4 h). The micronized tendon was transferred into a 200 L INOX reactor containing 140 L of distilled water, together with 5.88 g of Merck pepsin at 700 U FIP/mg and hydrochloric acid q.s. at pH 2.5. Agitation was maintained for 2- 4 h and the suspension was then kept at rest overnight at pH 2.5.

The reaction mass was filtered by sieving at 25 mesh and the filtrate brought very slowly to pH 5.5 with commercial sodium hydroxide 30%. The collagen fibres precipitated and were washed 3- 4 times with distilled water at pH 5.5 up to a chloride concentration of < 50 ppm.

The collected and weighed fibres were then dispersed in an aqueous solution of 0.3% acetic acid until a collagen concentration of 2% expressed as dry residue. 12.3kg of gel were obtained.

**Example 4. Preparation of membranes from collagen gel.**

The gel obtained according to the previous examples was stratified in trays previously charged with electrostatic current and air dried at 26°C for 56 h. The electrostatic charge was checked by an Ionizing Fan (RPO Electronic snc, Milan) working at primary volt 220, secondary volt 6000 VA power 50 Hz 50/60.

Membranes named A, B, E, were obtained which were sterilized by γ rays (gamma) at 25 KGy. The membranes thus produced were subjected to morphological analysis, tests of degradability by collagenase, permeability to water vapour according to the standard ASTM-E 96-90, and tensile tests according to conventional processes.

The membranes A, B, E were shown to have 3, 6, 9 mg/cm$^2$ of dry residue.

Morphological analysis

An initial characteristic of the membranes thus obtained was also detectable to the naked eye: in fact, also to the naked eye the sheets showed two different surfaces, an opaque side and a shiny one. This different superficial morphology was better characterized by observation both with the stereomicroscope and the Scanning Electron Microscope (SEM).

The analysis was carried out on 2 x 2 cm side samples cut out from the original sheets. The specimens were put as such under a stereomicroscope (Nikon, Japan).

For SEM analysis the sample was treated as follows: 2 small portions of material (approx. 5 mm x 3 mm) were cut out from each sample. These were placed on a special metallic support (stub) exposing for each sample (A, B and E) the shiny part and the opaque one (marked with a light incision on the support). The samples were covered with a palladium gold film to render its surface conductive and allows its observation under the SEM.

SEM analysis of the surfaces of the three samples highlighted the presence of fibres arranged at macromolecular level in the shiny part (see figure 1), while, in the opaque part, some superficial irregularities, protuberances and "bubbles" were visible giving a certain porosity to the membrane (see figure 2).

The thickness of the samples, measured with a digital micrometer, is shown in Table 1.

Table 1

| Sample A mm | Sample B mm | Sample E mm |
|---|---|---|
| 0.046 | 0.081 | 0.106 |
| 0.039 | 0.056 | 0.082 |
| 0.044 | 0.068 | 0.086 |
| 0.036 | 0.067 | 0.094 |
| 0.056 | 0.057 | 0.092 |
| 0.048 | 0.062 | 0.112 |
| 0.035 | 0.065 | 0.101 |
| 0.028 | 0.051 | 0.117 |
| Mean Values: 0.041 | 0.063 | 0.099 |
| σ* values 0.0087 | 0.0092 | 0.0124 |

* σ: standard deviation

Degradability test in the presence of collagenase

The collagen membranes batched P323 and P 50027, prepared as described in the previous examples and commercially available collagen membranes, were subjected to enzymatic degradation with bacterial collagenase (EC 3.4.24.3 from Clostridium Histolyticum I, 310 U/mg Sigma C-0130) 50 μg/ml, for 24 h in buffer at 37°C, for a comparative assay. Concentrations in μg/ml/24h of hydroxyproline hydrolyzed by collagenase were measured in the bath with a colorimetric method based on p-dimethylamminobenzoaldheyde.

Figure 3 shows that the samples P323 and P50027, obtained as described in the previous examples, are not degraded by the collagenase enzyme after 24 hours, while other commercial products namely Chondro-gide and Antema, are considerably degraded already after 24 hours, indicating that the membranes prepared according to the present invention are more resistant to degradation.

Permeability to water vapour

The tests were carried out on the samples (A, B, E) obtained as indicated in the previous examples, according to the so-called "water" test as described in the "Standard test method for water vapour transmission of materials," provided for by the standard ASTM E 96-90.

The WVT (water vapour transmission) values were obtained in the test according to the formula:
WVT= (G/t)/A
where:

A= area of the membrane;
G= variation in weight of the water in the measurement chamber, (grams)
t= time (hours),

and are shown together with the values of permeance, permeability and thickness of membranes in Table 2:

Table 2

| Sample | G/t (g/h) | WVT (g∗h$^{-1}$∗m$^{-2}$) | Permeance⊗ (g∗Pa$^{-1}$∗h$^{-1}$∗m$^{-2}$) | Permeability (g∗Pa$^{-1}$∗h$^{-1}$∗m$^{-1}$) | Thickness (mm) |
|---|---|---|---|---|---|
| A | 0.201 | 66.6105 | $7.138 \times 10^{-3}$ | $0.7138 \times 10^{-11}$ | 0.01 |
| B | 0.200 | 66.2791 | $7.102 \times 10^{-3}$ | $2.9828 \times 10^{-11}$ | 0.042 |
| E | 0.205 | 69.9361 | $7.494 \times 10^{-3}$ | $4.4964 \times 10^{-11}$ | 0.062 |

The permeance was calculated using the following formula:

$$\otimes \text{ Permeance} = WVT/ S (R1- R2)$$

and for the conditions adopted:
S = 46.66 x 10$^2$ Pa and R1- R2 = 0.5 (value expressed as a number fraction)
The mean permeability was calculated from the permeance values using the following formula:

$$\text{Permeability} = \text{permeance} \times \text{membrane thickness}$$

From table 2 it is seen that the permeability is directly proportional to the thickness of the membranes and besides that the permeability values of membranes prepared as described in the previous examples, are between $0.7138 \times 10^{-11}$ and $4.4946 \times 10^{-11}$ (g∗ Pa-1∗ h-1∗ m-1) for membranes with a thickness of between 0.01 and 0.062 mm.

Tensile strenght tests

From the original membranes prepared according to the present process, "dog bone" (three different batches) shaped specimens were cut out, of maximum length 76.7 mm, the thinnest part 3-6 mm wide and 22 mm long. The tensile tests were carried out with Chantillon apparatus. mod HTC (USA), according to conventional processes.
The values shown in table 3 were obtained.

Table 3:

| tensile strenght test | | |
|---|---|---|
| **Sample** | **Thickness mm** | **Load at break kg** |
| A1 | 0.04 | 0.4 |
| A2 | 0.03 | 0.4 |
| A3 | 0.03 | 0.5 |
| A4 | 0.03 | 0.25 |
| A5 | 0.015 | 0.5 |
| A6 | 0.02 | - |
| **Mean** | **0.0275** | **0.341** |
| B1 | 0.07 | 3.1 |
| B2 | 0.06 | 3.0 |
| B3 | 0.07 | 3.1 |

Table 3: (continued)

| tensile strenght test | | |
|---|---|---|
| Sample | Thickness mm | Load at break kg |
| B4 | 0.08 | 4.1 |
| **Mean** | **0.07** | **3.325** |
| E1 | 0.11 | 5.0 |
| E2 | 0.12 | 4.5 |
| E3 | 0.12 | 4.5 |
| **Mean** | **0.113** | **4.66** |

It can be demonstrated that the membrane thickness is correlated to its load at break with a logarithmic relationship (see Figure 4) described by the following equation:

$$y= a \log (x)+ b$$

In particular, for the mean values shown in the table, the value $\underline{a}$ is 7.08 and the value $\underline{b}$ is equal to 11.42 and the equation therefore becomes:
y= 7.08 x + 11.42, with $R^2$= 0.999
as measured for film thickness values comprised between 0.01 and 0.062 mm.

**Example 5: Preparation of membranes from collagen gel**

3 Kg of 2% collagen gel, prepared according to example 4, were diluted 1:2 with 0.3% acetic acid and shaken in a Rayneri mixer (endowed with sharp, curved blades, and rotating working at 1300 rpm) for 1.5 hours. They were then transferred into a two-speed prototype planetary mixer, (dedicated), in the vacuum (76 cm Hg) and therein kept for 2.5 hours for degassing.
The gel was then poured into trays made of melaminic plastic material, of size 25.5 x 16.5 cm or other size, previously charged with electrostatic current, at the thickness of 1.3 cm gel or other thickness in relation to the resistance that one wishes to give to the membrane.
The trays were put in an incubator on strictly levelled shelves and subjected to low ventilation of sterile air for 96h. The incubator works with a controlled no. of changes/h, and was set to 26 °C.
The air flow intake was diaphragmed so as not to brush the trays of collagen gel in any way and the internal geometry of the incubator was such as to prevent the formation of air vortices.
The trays are previously electrostatically charged by rubbing. The actual presence of electrostatic charge was checked by an Ionizing Fan (RPO Electronic snc, Milan) working at primary volt 220, secondary volt 6000 VA power 50 Hz 50/60. The chemical-physical analysis of the batch of membranes prepared according to the process gave the following values:

Exsiccation loss =          11.72% o.d.b. (on a dry bases)
Total hydroxyproline =     12.39% o.d.b.
Total nitrogen =              15.80% o.d.b.
Sodium =                       0.28% o.d.b.
Chlorides =                    0.19% o.d.b..
Sterility =                      sterile

**Example 6. Growth of fibroblasts on collagen membranes.**

The study was performed on human fibroblasts at the 2nd-3rd passage in culture. The cells were obtained from parodontal ligament.
At the beginning of experimentation the cells were elongated in parallel alignment as characteristic of fibroblast cells (sample OP) or in randomized alignment (sample B), or they were star-shaped in randomized alignment in sample A and C while replicative capacity was as in the control.

The following membrane batches were compared:

Sample A: membrane of type I collagen from bovine Achilles tendon, cross-linked with formaldehyde (BIOMEND, Collatech);

Sample B: membrane of type I collagen and of Chondroitin-4-sulphate, extracted from bovine skin, cross-linked with diphenylphosphorylazide (PAROGUIDE, Celetica);

Sample C: sponge of type I collagen from bovine skin not cross-linked, (GINGISTAT, Coletica);

Sample OP: membrane of type I collagen from equine Achilles tendon, produced according to that described in example 5;

Small 1x1cm squares of each membrane were placed in wells of NUNC plates together with 20000 cells per well in PBS (Dulbecco' Phosphate Buffered Saline). The suitable growth medium was then added to the wells and the cells were then incubated at 37°C; Platelet Growth Factor BB (PGF-BB) 50 ng/ml (Boehringer M.) was also added to the culture medium in some tests. The control consisted only of cells without membrane.

At the end of the experiment (72 hours) all the samples were trypsinized (incubation in 200 µl of 0.25% trypsin for 5') so as to detach the cells from the various substrates and make them available for counting. The samples were also observed under the optic microscope and SEM before trypsinization.

The following data was then collected:

1) Cell count: number of total cells present in the well at 72 hours:

| | |
|---|---|
| Control of A | 22000 |
| Control+ PGF-BB | 24750 |
| Sample | 0 (<5000) |
| Sample A+ PGF-BB | 0 (<5000) |
| Control of B, C, OP | 21000 |
| Control+ PGF-BB | 36000 |
| Sample B | 10500 |
| Sample B + PGF-BB | 14000 |
| Sample C | 0 (<5000) |
| Sample C + PGF-BB | 0 (<5000) |
| Sample OP | 8250 |
| Sample OP + PGF-BB | 5500 |

2) Optical microscopy: the cell growth in the wells and onto the membranes was observed under the optic microscope.

Sample A: few cells of anomalous morphology, an indication of cellular suffering, more apparent the shorter the distance from the membrane.

Sample B: elongated and adhered cells present at a distance from the membrane. Non-adherent spherical cells near the membrane.

Sample C: elongated and adhered cells present at a distance from the membrane. Non-adherent spherical cells near the membrane

Sample OP: elongated and adhered cells present at a distance from the membrane. Non-adherent spherical cells near the membrane

3) Electronic Microscopy: observations of membranes under the SEM

Sample A: membrane essentially without fibroblasts: however, the very unusual cellular elements present show normal morphology.

Sample B: no cell on the membrane.

Sample C: no cell on the membrane.

Sample OP: cells of normal morphology present on the membrane.

From data relating to the cell count (assay (1)) after trypsinization it is seen that the OP membrane reduces only slightly the cellular growth with respect to the control, while samples A and C completely abolish cellular growth(<5000, lower detection limit of the method) . Sample B made up of collagen and chondroitin-4-sulphate, also allows cellular proliferation, as shown in the cell count test.

However, it is also seen from the analyses under the optic and electron microscope that in the sample where marked inhibition of cellular growth (B) was not found, colonization of the membrane itself was absent since the fibroblasts present were all arranged around the membrane, but not on its surface.

The only membrane colonized by the fibroblasts as observed by SEM is the OP 323 membrane produced according to the process described in the previous examples. From the morphological point of view, the cells grown on the OP membrane as observed by optical microscopy, looked healthy.

**Example 7: Preparation of membranes batch 40046.**

500 g of horse tendon (batch 40041) were treated exactly as described in example 3, until the attainment of collagen fibers isolated by salting out (precipitation) at pH 5.5.

10 kg of said fibers were treated with 20 l of 1 N NaOH and left under agitation for 1 hour at room temperature then washed and brought up to pH 5.5 and at a chloride concentration of ≤ 50 ppm.

This treatment with 1 N NaOH besides representing compliance with an EEC directive for the purposes of the removal of potential viral contaminants and agents responsible for TSE (however much this pathology has so far never been found in horses), has led, as shown in the following examples, by the comparative and microscopic analyses, to greater purity of the collagen and presumably to a modest reduction in glycosilation. In table 4 are shown the results of the analyses performed on the membranes before (A) and after (B) NaOH treatment.

Table 4

|  | A | B |
|---|---|---|
| Hydroxyproline (d.s.) | 13.14 % | 13.39% |
| Nitrogen (d.s.) | 17.86% | 17.74% |
| Sodium (d.s.) | 0.129 | 0.067% |
| Chlorides (Cl⁻ on d.s.) | 0.646 | 0.679% |
| Hexosamine (on dry s.) | 0.737 | 0.181% |
| d.s.= on dry substance | | |

The collected and weighed fibres were dispersed in an aqueous solution of 0.3% acetic acid and at a presumed collagen concentration (as dry substance) of ~ 1 %. 9.48 kg of gel were obtained.

These 9.48 kg of collagen gel were set under agitation for 1.5 hours in a Rayneri Dynavar SB 25 mixer (Groupe VMI ZI, Montaign France), at speed n.80. The gel is then transferred in a suitable homogenizer (CONDOR S.r.l. Verona) equipped with a planetary mixer and kept in a vacuum and shaken for 3 hours at speed 1 and for 3 hours at speed 2. The gel is then left in the vacuum to rest overnight.

The gel, free from air bubbles included after this treatment, is divided into 12 x 12 cm small polystyrene trays charged with electrostatic current, in amounts of 130 g of gel/tray, corresponding to membranes with an expected weight of approx. 9 mg/cm2.

The trays were put in an incubator cabinet "High Performance" Mod 2800 (F.Ili Galli G.& P.- Milan) thermostatically set at 28°C without ventilation and therein left for approx. 120 h.

Upon exit from the exsiccator cabinet the films formed are extracted from the trays and left in contact with air for 1 hour then packed in a sterile bag. Afterwards the membranes were cut into suitable shapes, packed in a double bag and finally subjected to radiant treatment with γ rays at the dose of 25 Kgy.

| Analysis of the membranes of batch 40046 provided the following composition: | |
|---|---|
| Humidity | 15.6.% |
| Acetic acid content, measured by potentiometry | 2.5% |
| Content in hydroxyproline | 13.39% |

Upon enzymatic digestion with collagenase the liberation kinetics of hydroxyproline, in the 40046 membranes, measured as shown in example 4 (with p-dimethylaminobenzoaldehyde), proved perfectly comparable with that of collagen membranes prepared as in example 4, i.e. without treatment with sodium hydroxide.

**Example 8: preparation of membranes batch 40046/LL**

Following the removal of the residual acetic acid by washing, some of membranes 40046, prepared as described in example 7, were cut into four parts and placed in distilled water, which was changed 4 consecutive times after 1 hour immersion each time.

The membranes were exsiccated by lyophilization with at a temperature ranging from -30°C to +30°C.

Membranes marked 40046/LL were obtained, which showed a wrinkled superficial appearance, basically curled and scarcely hydrophilic, characteristic of a superficially denatured proteic structure.

Analysis provided the following percentages:

Humidity: 13.5%

Acetic acid content: ≤ 0.23%

Upon Differential Scanning Calorimetry (DSC) analysis, the thermal denaturation of 40046LL occurred at the temperatures $t_{start}$ 49.62°C, $t_{max}$ 56.09°C, and $t_{end}$ 62.17, against the respective values of membrane 40046 of 43.74°C, 49.52°C and 55.29°C, while the apparent heat capacity was 6.09 cal/g for membrane 40046/LL, compared to 2.82 cal/g for the unwashed/lyophilized membrane 40046.

Membranes 40046 and 40046/LL were also subjected to the absorption and water evaporation test.

The membranes 40046 absorbed 9% of their weight of water in 4 hours, while membranes 40046/LL absorbed only 4.3%. On the other hand, the latter gave up the water absorbed with a speed 40% lower than that of water evaporation of membranes 40046.

These results indicate that the process of lyophilization is not suited for the collagen membranes as it partly degrades the proteic structure of the superficial collagen.

**Example 9. Preparation of the membranes 40036.**

The membranes 40036 were prepared as the 40046 ones (example 7) with the only exclusion of the alkaline treatment of collagen fibers with 1N NaOH.

**Example 10. Preparation of membranes P322.**

1% collagen gel batch n. 78631 is prepared from equine Achilles tendon, as described in example 3. 2 kg of said gel are put in a 10 litre vacuum flask for 60' for deaeration, then it was divided in 90 mm ∅ plates so as to be able to have a membrane of approx. 16 mg of dry residue/cm$^2$. The plates were air dried by spontaneous incubator exsiccation. All the operations were carried out in under controlled microbial charge. Batch 88225 of membranes marked P322 were obtained.

**Example 11:Preparation of membranes 40049A/S largely consisting of collagen type II**

Ground pig's trachea (batch RD/7) 0.9 kg in 5 l of 0.5M NaOH shaken for 5 h at room temperature then at 5°C. The fragments of fatty and/or phospholipidic material were mechanically eliminated from the supernatant.

The residue was collected on a filter consisting of a fine metallic net, washed with water 3 x 2 litres and neutralized at pH 7 with 80 ml of 1N HCl.

The mass was twice treated with 2 l of acetone each time. The hydroacetonic step is eliminated, the mass is washed with water, added with 14 l of water 30 ml of 1N HCl up to pH 2.6 and 90g of pepsin from pig's gastric mucous, Merck at 700FIP U/g (batch 15506) and left under slow agitation for 24 h at 20-24°C.

5.5 l of said suspension were vacuum filtered, on buckner with octex® filters and pre-stratum of filtering aid. The filtrate, opalescent, was brought to pH 3.5 with HCl, treated with NaCl up to 0.7M and left to rest overnight.

The light precipitate, consisting of type II collagen fibers was collected by centrifugation. 80 ml of damp fibers were obtained and marked 40049A.

80 ml of fibers 40049° are put in a Spectrapor MWCO 1000 dialysis tube ∅ 4.5 and length 70 cm, and therein left for 48h at room temperature with periodic changes of water.

The retented, approx. 100 ml is brought to pH 3.36 with 50% HCl solution. The gel obtained (1.18% dry residue) was divided into polystyrene petri capsules, ∅ 5.5 cm, so as to give rise to 9 mg/cm2 membranes, and dried first for 30 h in a stove then in air on plates at 30°C.

The resulting membranes were marked 40049A/S.

**Example 12. Analysis of collagen film under the optic microscope, scanning and transmission electron microscope**

The collagen membranes marked 40039/E or 40046, prepared as described in the examples previously described, were washed in sterile distilled $H_2O$, for approx. 15 hours, then subjected to analysis by optic, scanning electron and transmission microscopy.

Semi-fine sections, perpendicular to the membrane surface, were stained with basic dyes of the thiazine group and observed under the optic microscope. One surface (most probably the upper one) is smooth, compact and intensely coloured; the lower surface is less homogeneous, more loose and uneven (Figure 5).

Under the *scanning electron microscope,* the membrane appeared to be formed of a three-dimensional lattice of collagen fibrillae, most of which set in longitudinal bundles parallel to the gel surface. One side of the gel, most probably the lower one, has longer fibrillae and which are arranged in a more uniform way as regards the other surface (upper), which overall is more wrinkled and dishomogeneous (Figure 6).

Under the *transmission electron microscope,* the collagen fibrillae, of varying dimensions, form a three-dimensional lattice, with predominant orientation in a parallel direction to the gel surface. The texture is, in some points, quite regular.

Most of the collagen fibrillae are linear, some are folded over and the arrangement is not always clear. As mentioned, the diameter of the fibrillae is very variable, while the periodic arrangement, when recognizable, is 640 nm as for native collagen.

**Example 13: Measurement of the growth of dermal fibroblasts cultivated on collagen film**

Human dermal fibroblasts were used, isolated from skin biopsies of healthy adult subjects, cultivated in Dulbecco's Modification of Eagle's Medium (DMEM) culture medium at high glucose concentration, containing 2mM L-glutamine, 100 IU/ml penicillin, 100 mg/ml streptomycin, 1 mM Na pyruvate, non-essential amino acids (NEAA) 1X and fetal bovine serum (FBS) at a final concentration of 10%.

In this assay the fibroblasts were plated in a two well "chamber slide" (area= 8.26cm$^2$), 30,000 cells/well in a final volume of 2 ml culture medium.

The cells were directly plated in one well, in the other before plating, was put the collagen film with the opaque side turned upwards.

The MTT test (tetrazolium salts) was used to determine the growth of cells.

This test is based on the intracellular reduction of the tetrazolium safts, by the mitochondrial dehydrogenase, in a product of brick red colour insoluble in water (formazan crystals). The crystals are solubilized with dimethyl sulphoxide and the coloured solution obtained is measured using the spectrophotometer at a wavelength of 565 nm.

The vital cells, unlike the dead ones, reduce the tetrazolium salts. This test is much used for measuring cellular vitality and as an indicator of proliferation.

The number of fibroblasts, grown on the plastic well adapted for cell-growth, approximately double by the 4th day of culture with respect to the 1st plating day.

The fibroblasts grown on the collagen film show the same tendency (Figure 7).

**Example 14. Analysis of dermal fibroblasts cultivated on collagen film under the confocal microscope**

To check the morphology of the cells cultivated on the collagen membranes and the level of growth, after 3 and 6 days of culture, the collagen membranes with the fibroblasts were fixed and stained with fluorescinated phalloidine, that emits green radiation and highlights the cytoskeleton of actin and with rhodaminated tubulin that emits red radiation and highlights the tubulin structures.

After staining, the membranes with the fibroblasts, were examined under the fluorescence optic microscope and the confocal microscope.

The cells cultivated on the collagen membrane were clearly visible and appeared to be distributed on different levels of the film.

After 3 days, the cells still had not reached confluence. They appeared heterogeneous: elongated with thin polygonal, triangular protrusions. In certain fields mitotic cells were observed.

At day 6, the fibroblasts, of polygonal shape and elongated, were at confluence, they covered all the membrane space (Figure 8 fibroblasts grown on collagen film at the 6th day of culture (labeling with Phalloidine) Obj: 10.0 Zoom: 1.430)

**Example 15. Growth, proliferation and evaluation of the vitality of chondrocytes taken from knee cartilage of patients undergoing prosthesis surgical treatment.**

Collagen membranes batch 40046. prepared as described in example 7, were used in an experimental scheme suited to assess the growth, proliferation and vitality of human chondrocytes taken from knee cartilage of patients undergoing surgical treatment for prosthetic implant.

The cultures were grown in autologous serum and were examined at 0, 1, 7, 14, 21 and 35 days. The growth and vitality of the cells were tested by MTT assay, 3-(4.5-dimethyltiazol-2-yl)-2.5-diphenyl tetrazolium bromide reduced into formazan by mitochondrial enzyme succinate-dehydrogenase according to Denizot F., Lang R., J Immunol Methods, 89, 271-277,1986.

After 7 days it was surprisingly found that not only are the cells vital and healthy on the membranes in culture but that they grew more than was to be expected. Due to their impressive growth on the membranes, it was difficult for the operator to assess their proliferation degree, as they have already reached the confluence point very early.

In order to evaluate chondrocytes growth and vitality for 35 days, the protocol had to be modified: instead of plating 1.000.000 cells/cm$^2$, they had to plate membranes 40046 with 250.000 cells/ cm$^2$.

After this modification, the same healthy morphology and the same unexpected proliferation rate was observed after 35 days in colture.

The extremely positive results both on fibroblasts and on chondrocytes might be, at least in part, due to the additional alkaline treatment and to the additional precipitation step of the collagen fibres introduced in the preparation process.

**Example 16. Comparative assays to determine human chondrocytes proliferation and vitality on some collagen membranes**

1x10$^4$ chondrocytes from human cartilage have been cultured for 7 days in parallel on the following membranes:

- 40046, prepared as described in example 7
- 40046LL, prepared as described in example example 8
- 40036, prepared as described in example example 9
- P322, prepared as described in example 10
- 40049/A/S, prepared as described in example 11
- Chondro-gide, Geistlich

At the end of the estabished period the membranes colonized by cells have been colored with Safranine-O, that highlights the characteristic sulphate group of the cartilagineous extracellular matrix synthetized by vital chondrocytes. Histomorphometry of the cultivated chondrocytes on the membranes has been evaluated by analysing the images and representing the results as area of colonized part of the membrane expressed as percentage of the entire membrane.

The hystogram shows that the best membranes, among those examined, are 40049/A/S and 40046. In particular, membrane 40046 has been chosen for other studies because made with equine collagen Type I already characterized from the immunological and toxicological point of view (see Bianchini P., Parma B., Ameim-Forsh 51(l, 414-419, 2001). In figure 10 and 11 are shown the optical microscopy pictures related to chondrocytes colonization of two different membranes: Chondro-gide and membrane 40046.

**Claims**

1.  Double sided collagen membranes with a porous side suitable for the adhesion and/or growth of cells and a smooth side, **characterized in that** the collagen is arranged at macromolecular level on the smooth side, obtainable by applying an electric field or an electrostatic charge to a collagen gel.

2.  Membranes according to claim 1 wherein said collagen is chosen among: collagen of type I, II, III or IV, or a mixture consisting of at least two of the four types.

3.  Process for the preparation of the collagen membranes according to claim 1 **characterized in that** a collagen gel is poured and allowed to exsiccate on a plate on which an electrostatic charge has been induced.

4. Process according to claim 3, wherein said collagen gel has a collagen concentration comprised between 0.5-6%

5. Process according to claim 3 wherein said exsiccation of the gel occurs at a temperature of less than 40°C, for a time of at least 40 hours.

6. Process according to claim 4, wherein said exsiccation temperature is between 20 and 30°C.

7. Process according to claim 4 wherein said exsiccation is performed in the absence of any air turbulence

8. Process according to claim 3, wherein said collagen gel is produced with a process that essentially comprises the following steps:

> a) mechanical mincing of a connective tissue and homogenization into a collagen suspension,
> b) treatment of the collagen suspension with proteolytic enzymes,
> c) alkaline treatment of the collagen suspension to a pH value of at least 8 and neutralization with a strong acid to a pH comprised between 5 and 6,
> d) precipitation of the collagen fibers by salting out
> e) resuspension of the precipitate in an aqueous solution diluted with a weak acid and obtainment of a collagen gel.

9. Process according to claim 8, wherein the following additional steps are added in between step d) and e):

> d') resuspension of the precipitate in a strong alkaline solution with mixing for a time of at least 30',
> d") precipitation of the collagen fibers by salting out of the alkaline solution at pH values comprised between 5 and 6

10. Process according to claim 9, wherein said alkaline solution is 1N NaOH

11. Process according to claim 10, wherein said time is comprised between 45' and 75'

12. Process according to claims 9 or 10, wherein the connective tissue of step a) is derived from a mammalian.

13. Process according to claims 8 or 9, wherein the connective tissue of step a) is tendon.

14. Process according to claim 13, wherein said tendon is equine.

15. Process according to claims 8 or 9, wherein said connective tissue in step a) is a tracheal tissue.

16. Process according to claim 15, wherein said tracheal tissue is derived from a bovine, a swine or an equine.

17. Process according to claim 8 or 9 wherein mincing and homogenization in step a) occurs after the addition of an aqueous solution and up to the attainment of a suspension consisting of collagen particles less than 25 mesh in size.

18. Process according to claim 17 wherein the homogenization is followed by micronization.

19. Process according to claims 8 or 9 wherein the proteolytic enzyme in step b) of the process is pepsin and the treatment occurs at a pH comprised between 2 and 3 for a time comprised between 10 and 20 hours.

20. Process according to claims 8 or 9 wherein the resuspension of the collagen precipitate in step e) is performed at a collagen concentration comprised between 0.5 and 6%.

21. Process according to claim 20 wherein said concentration is between 2 and 3%.

22. Process according to claims 8 or 9 wherein said diluted aqueous solution, as in step (e) of the procedure, is a solution of acetic acid.

23. Process according to claim 22 wherein said acetic acid solution has a concentration comprised between 0.1 and 1% (w/V).

24. Process according to claims 8 or 9, wherein the collagen gel obtained in step e) of the process is further homogenized and degassed by aspiration in anyone of the vacuum.

25. Collagen membranes obtainable by the process according to claims 3-7.

26. Use of the collagen membranes according to claims 1 or 25 as supports for the *in vitro* adhesion, growth or colonization of mammalian cells.

27. Use of the collagen membranes according to claim 26 wherein said mammalian cells are selected from: staminal cells, fibroblasts, epithelial cells, endothelial cells, osteocytes and chondrocytes.

28. Membranes according to claims 1 or 25 for use in therapy

29. Use of the membranes according to claim 28 for the preparation of cellular matrices for tissue reconstruction

30. Use according to claim 29 wherein said tissue reconstruction is chosen among: reconstruction of epithelial, endothelial, connective, cartilaginous, osseous and osteo-cartilaginous tissue.

31. Membranes according to claim 28 for tissue reconstruction in chronic degenerative diseases.


**Patentansprüche**

1. Doppelseitige Kollagenmembranen mit einer porösen Seite für die Adhäsion und/oder die Anzucht von Zellen und einer glatten Seite, **dadurch gekennzeichnet, dass** das Kollagen auf dieser glatten Seite auf makromolekularer Ebene angeordnet ist und durch Anlegen eines elektrischen Feldes oder einer elektrostatischen Ladung am Kollagengel erhalten wird.

2. Membranen gemäß Anspruch 1, wobei das genannte Kollagen ausgewählt wird unter: Kollagen vom Typ I, II, III oder IV oder einer Mischung enthaltend mindestens zwei der vier Typen.

3. Verfahren zur Herstellung der Kollagenmembranen gemäß Anspruch 1, **dadurch gekennzeichnet, dass** ein Kollagengel auf eine Platte, an der eine elektrische Ladung induziert wurde, gegossen und zum Trocknen belassen wird.

4. Verfahren gemäß Anspruch 3, wobei das genannte Kollagengel eine Kollagenkonzentration zwischen 0,5-6% aufweist.

5. Verfahren gemäß Anspruch 3, wobei genannte Trocknung des Gels bei einer Temperatur von unter 40 °C über einen Zeitraum von mindestens 40 Stunden erfolgt.

6. Verfahren gemäß Anspruch 4, wobei die genannte Trocknungstemperatur zwischen 20 °C und 30 °C liegt.

7. Verfahren gemäß Anspruch 4, wobei genannte Trocknung ohne Luftströmung durchgeführt wird.

8. Verfahren gemäß Anspruch 3, wobei genanntes Kollagengel mittels eines Verfahrens hergestellt wird, welches folgende Schritte umfasst:

   a) Mechanische Zerkleinerung von Bindegewebe und Homogenisierung bis zu einer Kollagensuspension,
   b) Behandlung der Kollagensuspension mit proteolytischen Enzymen,
   c) Alkalische Behandlung der Kollagensuspension bis zum Erreichen eines pH-Wertes von mindestens 8 und Neutralisation mit einer starken Säure bis zu einem pH-Wert zwischen 5 und 6,
   d) Präzipitation der Kollagenfasern durch Aussalzen,
   e) Resuspension des Präzipitates in einer wässrigen Lösung verdünnt mit einer schwachen Säure und Gewinnung von Kollagengel.

9. Verfahren gemäß Anspruch 8, wobei folgende Zusatzschritte zwischen den Schritten d) und e) ausgeführt werden:

d') Resuspension des Präzipitates in einer starken alkalischen Lösung unter Rühren für einen Zeitraum von mindestens 30 Minuten,

d")Präzipitation der Kollagenfasern durch Aussalzen der alkalischen Lösung bei pH-Werten zwischen 5 und 6.

**10.** Verfahren gemäß Anspruch 9, wobei es sich bei der genannten alkalischen Lösung um IN NaOH handelt.

**11.** Verfahren gemäß Anspruch 10, wobei genannte Zeit zwischen 45 und 75 Minuten beträgt.

**12.** Verfahren gemäß Anspruch 9 oder 10, wobei das Bindegewebe von Schritt a) von einem Säugetier stammt.

**13.** Verfahren gemäß Anspruch 8 oder 9, wobei es sich bei dem Bindegewebe von Schritt a) um Sehnengewebe handelt.

**14.** Verfahren gemäß Anspruch 13, wobei das genannte Sehnengewebe vom Pferd stammt.

**15.** Verfahren gemäß Anspruch 8 oder 9, wobei es sich bei dem Bindegewebe von Schritt a) um Gewebe aus der Trachea handelt.

**16.** Verfahren gemäß Anspruch 15, wobei das Trachealgewebe vom Rind, Schwein oder Pferd stammt.

**17.** Verfahren gemäß Anspruch 8 oder 9, wobei das Zerkleinern und Homogenisieren von Schritt a) nach der Zugabe einer wässrigen Lösung und bis zum Erreichen einer Suspension bestehend aus Kollagenpartikeln mit einer Größe unter 25 Mesh erfolgt.

**18.** Verfahren gemäß Anspruch 17, wobei auf die Homogenisierung eine Mikronisierung folgt.

**19.** Verfahren gemäß Anspruch 8 oder 9, wobei es sich bei dem proteolytischen Enzym aus Schritt b) des Verfahrens um Pepsin handelt und die Behandlung bei einem pH-Wert zwischen 2 und 3 durchgeführt wird über einen Zeitraum zwischen 10 und 20 Stunden.

**20.** Verfahren gemäß Anspruch 8 oder 9, wobei die Resuspension des Kollagenpräzipitates in Schritt e) bei einer Kollagenkonzentration zwischen 0,5 und 6% durchgeführt wird.

**21.** Verfahren gemäß Anspruch 20, wobei die genannte Konzentration zwischen 2 und 3% liegt.

**22.** Verfahren gemäß Anspruch 8 oder 9, wobei genannte verdünnte wässrige Lösung, wie in Schritt (e) des Verfahrens eine Essigsäurelösung ist.

**23.** Verfahren gemäß Anspruch 22, wobei genannte Essigsäurelösung eine Konzentration zwischen 0,1 und 1% (w/V) besitzt.

**24.** Verfahren gemäß Anspruch 8 oder 9, wobei das in Schritt e) des Verfahrens erhaltene Kollagengel weiter homogenisiert und durch Anlegen eines Vakuums entgast wird.

**25.** Kollagenmembranen erhältlich durch das Verfahren gemäß einem der Ansprüche 3-7.

**26.** Verwendung der Kollagenmembranen gemäß Anspruch 1 oder 25 als Träger für die In-Vitro-Adhäsion, Anzucht oder Ansiedelung von Säugetierzellen.

**27.** Verwendung der Kollagenmembranen gemäß Anspruch 26, wobei genannte Säugetierzellen ausgewählt werden unter: Stammzellen, Fibroblasten, Epithelzellen, Endothelzellen, Osteozyten und Chondrozyten.

**28.** Membranen gemäß Anspruch 1 oder 25 zur Verwendung in der Therapie.

**29.** Verwendung der Membranen gemäß Anspruch 28 zur Herstellung von Zellmatrices für die Geweberekonstruktion.

**30.** Verwendung gemäß Anspruch 29, wobei für die genannte Geweberekonstruktion ausgewählt wird unter: Rekonstruktion von Epithel-, Endothelgewebe, Bindegewebe, Knorpel-, Knochen- und Osteochondralgewebe.

**31.** Membranen gemäß Anspruch 28 zur Geweberekonstruktion bei chronisch degenerativen Erkrankungen.

**Revendications**

**1.** Membranes de collagène double face avec un côté poreux approprié à l'adhérence et/ou la croissance de cellules et un côté lisse, **caractérisées en ce que** le collagène est agencé au niveau macromoléculaire du côté lisse, que l'on peut obtenir par application d'un champ électrique ou d'une charge électrostatique et un gel de collagène.

**2.** Membranes selon la revendication 1, où ledit collagène est choisi parmi: collagène du type I, II, III ou IV ou un mélange consistant en au moins deux des quatre types.

**3.** Procédé pour la préparation des membranes de collagène selon la revendication 1 **caractérisé en ce qu'**un gel de collagène est versé et on laisse s'assécher sur une plaque sur laquelle a été induite une charge électrostatique.

**4.** Procédé selon la revendication 3, où ledit gel de collagène a une concentration en collagène comprise ente 0,5 et 6%.

**5.** Procédé selon la revendication 3, où l'assèchement du gel se produit à une température de moins de 40°C pendant un temps d'au moins 40 heures.

**6.** Procédé selon la revendication 4, où la température d'assèchement est comprise entre 20 et 30°C.

**7.** Procédé selon la revendication 4, où ledit assèchement est accompli en l'absence de toute turbulence d'air.

**8.** Procédé selon la revendication 3, où ledit gel de collagène est produit avec un procédé qui comprend essentiellement les étapes suivantes:

a) la mise en hachis mécanique d'un tissu conjonctif et l'homogénéisation en une suspension de collagène,
b) le traitement de la suspension de collagène avec des enzymes protéolytiques,
c) le traitement alcalin de la suspension de collagène à une valeur de pH d'au moins 8 et la neutralisation avec un acide fort jusqu'à un pH compris entre 5 et 6,
d) la précipitation des fibres de collagène par dessalage,
e) la remise en suspension du précipité dans une solution aqueuse diluée avec un acide faible et l'obtention d'un gel de collagène.

**9.** Procédé selon la revendication 8, où les étapes additionnelles qui suivent sont ajoutées entre l'étape d) et e)

d') la remise en suspension du précipité dans une solution alcaline forte avec mélange pendant un temps d'au moins 30',
d'') la précipitation des fibres de collagène par dessalage de la solution alcaline à des valeurs de pH comprises entre 5 et 6.

**10.** Procédé selon la revendication 9, où ladite solution alcaline est NaOH 1N.

**11.** Procédé selon la revendication 10, où ledit temps est compris entre 45' et 75'.

**12.** Procédé selon les revendications 9 ou 10, où le tissu conjonctif de l'étape a) est dérivé d'un mammifère.

**13.** Procédé selon la revendication 8 ou 9, où le tissu conjonctif de l'etape a) est du tendon.

**14.** Procédé selon la revendication 13, où ledit tendon est équin.

**15.** Procédé selon les revendications 8 ou 9, où ledit tissu conjonctif à l'Etape a) est un tissu de trachée.

**16.** Procédé selon la revendication 15, où ledit tissu de trachée est dérivé d'un bovin, d'un cochon ou d'un équin.

**17.** Procédé selon la revendication 8 ou 9, où la mise en hachis et l'homogénéisation à l'étape a) se produit après addition d'une solution aqueuse et jusqu'à l'atteinte d'une suspension consistant en particules de collagène d'une maille de moins de 25.

18. Procédé selon la revendication 17, où l'homogénéisation est suivie d'une micronisation.

19. Procédé selon les revendications 8 ou 9, où l'enzyme protéolytique de l'étape b) du procédé est la pepsine et le traitement se produit à un pH compris entre 2 et 3 pendant un temps compris entre 10 et 20 heures.

20. Procédé selon les revendications 8 ou 9, où la remise en suspension du précipité de collagène à l'étape e) est accomplie à une concentration en collagène comprise entre 0,5 et 6%.

21. Procédé selon la revendication 20, où ladite concentration est comprise entre 2 et 3%.

22. Procédé selon les revendications 8 ou 9, où ladite solution aqueuse diluée, comme à l'étape e) du procédé, est une solution d'acide acétique.

23. Procédé selon la revendication 22, où ladite solution d'acide acétique a une concentration comprise entre 0,1 et 1% (p/V).

24. Procédé selon les revendications 8-9, où le gel de collagène obtenu à l'étape e) du procédé est de plus homogénéisé et dégazé par aspiration sous vide.

25. Membrane de collagène pouvant être obtenue par le procédé selon l'une quelconque des revendications 3-7.

26. Utilisation des membranes de collagène selon les revendications 1 ou 25 en tant que supports pour l'adhérence, la croissance ou la colonisation *in vitro* de cellules mammaliennes.

27. Utilisation des membranes de collagène selon la revendication 26, où lesdites cellules mammaliennes sont sélectionnées parmi: cellules staminales, fibroblastes, cellules épithéliales, cellules endothéliales, ostéocytes et chondrocytes.

28. Membranes selon les revendications 1 ou 25 à utiliser en thérapie.

29. Utilisation des membranes selon la revendication 28 pour la préparation de matrices cellulaires pour la reconstruction des tissus.

30. Utilisation selon la revendication 29, où ladite reconstruction des tissus est choisie parmi: reconstruction du tissu épithélial, endothélial, conjonctif, cartilagineux, osseux et ostéo-cartilagineux.

31. Membrane selon la revendication 28 pour la reconstruction des tissus dans des maladies de dégénérescence chronique.

Fig. 1

Fig. 2

FIG. 3

y = 11.420 + 7.0806*LOG(x)   R^2 = 0.999

FIG. 4

EP 1 307 247 B1

Fig. 5

Fig. 6

Fig.7

Fig. 8

Fig. 9

Fig. 10

Fig. 11a

Fig. 11b